Europäisches Patentamt

European Patent Office

Office européen des brevets

(11) Publication number: **0 057 597**
**B1**

# EUROPEAN PATENT SPECIFICATION

(45) Date of publication of patent specification: **26.06.85**

(51) Int. Cl.⁴: **A 61 F 2/42**

(21) Application number: **82300486.6**

(22) Date of filing: **29.01.82**

(54) **A joint prosthesis.**

(30) Priority: **30.01.81 GB 8102977**

(43) Date of publication of application:
**11.08.82 Bulletin 82/32**

(45) Publication of the grant of the patent:
**26.06.85 Bulletin 85/26**

(84) Designated Contracting States:
**CH DE FR GB LI**

(56) References cited:
**DE-A-3 021 443**
**DE-B-2 444 668**
**DE-C-1 960 087**
**GB-A-1 320 956**
**US-A-3 462 765**

(73) Proprietor: **OEC EUROPE LIMITED**
**134 Brompton Road**
**London S.W.3. (GB)**

(72) Inventor: **Helal, Basil Hessein**
**Broomer Cottage Churchgate**
**Cheshunt Hertfordshire (GB)**

(74) Representative: **Burrows, Anthony Gregory**
**et al**
**Haseltine Lake & Co. Hazlitt House 28**
**Southampton Buildings Chancery Lane**
**London, WC2 1AT (GB)**

Courier Press, Leamington Spa, England.

## Description

This invention relates to a joint prosthesis, particularly a small joint spacing prosthesis usable in the hands and feet.

Various small joint prosthesis are known which are usable in the hands and feet. They are each designed to bend at a single location substantially centrally of their length. Some of them incorporate a spacer which maintains spaced apart the respective adjacent ends of the bones and thus prevents the intramedullary stems of the prosthesis from gradually penetrating further and further along the bones during use, which is particularly disadvantageous if the stems increase in thickness progressing towards the axis of articulation. Examples of prostheses incorporating such spacers are disclosed in United States Patent 3,593,342 and Federal German Offenlegungsschrift 3,021,443, but, since their spacers have to be thick at their ends to provide abutment shoulders for the bone ends and yet thin at their middles where the bending is to occur, these spacers can nip in-growing body tissue during bending, and this will limit movement in the joint. In order to overcome this problem, there is disclosed in British Patent 1320956 a small joint prosthesis in which the spacer is constituted by a resilient, deformable capsule enclosing the hinge of the prosthesis and incorporating abutment shoulders at its ends. This increases the complexity and cost of the prosthesis.

According to the present invention, there is provided a joint prosthesis, comprising a spacer for insertion between first and second bones, and first and second intramedullary stems extending from respective opposite ends of said spacer for insertion into the respective bones, the spacer being of greater width than the stems, thereby providing shoulders against which the respective ends of the bones can abut, characterized in that the arrangement is such that, in the prosthesis when implanted, bending of the joint occurs at the respective end zones of the stems nearer the spacer.

Owing to the present invention, it is possible to avoid nipping of in-growing body tissue at a central hinge and yet also keep the prosthesis relatively simple and inexpensive.

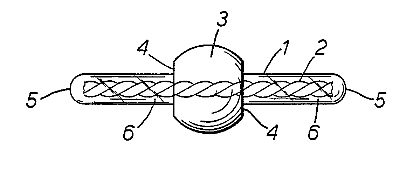

In order that the invention may be clearly understood and readily carried into effect, reference will now be made, by way of example, to the accompanying drawing, which shows an elevation of a joint prosthesis.

Referring to the drawing, the prosthesis is a small joint spacing prosthesis which is usable in the hands and feet and would in practice be provided in various sizes. It consists of an elongate silicone rubber body 1 reinforced by a Dacron or nylon string 2, and has been made by moulding the body 1 with the string 2 therein. The middle of the body 1 is of increased diameter to form a spacer 3 which is of spherical shape except that it has flat end faces 4. The remaining parts of the body 1 are of constant diameter except at their rounded ends 5 and form intramedullary stems 6 projecting centrally from the respective end faces 4.

During implantation the stems 6 are inserted into resectioned adjacent ends of respective bones and the spacer 3 positioned between the resectioned ends abutting against the end faces 4.

The prosthesis can be used in replacements of metacarpo-phalangeal joints, metatarso-phalangeal joints, proximal interphalangeal joints of the fingers, and the carpo-metacarpal joint of the thumb, for example. From a cosmetic viewpoint, in the metacarpo-phalangeal joint, the prosthesis reproduces the knuckle feature. Bending of the joint occurs at the two inner end zones of the stems 6. Because the bone ends abut the definite limits provided by the faces 4, there is no tendency for the stems 6 to penetrate more deeply into the medullae during use of the joint.

## Claims

1. A joint prosthesis, comprising a spacer (3) for insertion between first and second bones, and first and second intramedullary stems (6) extending from respective opposite ends of said spacer (3) for insertion into the respective bones, the spacer (3) being of greater width than the stems (6), thereby providing shoulders (4) against which the respective ends of the bones can abut, characterized in that the arrangement is such that, in the prosthesis when implanted, bending of the joint occurs at the respective end zones of the stem (6) nearer the spacer (3).

2. A prosthesis according to claim 1, wherein said stems (6) comprise elastomeric material.

3. A prosthesis according to claim 1 or 2, wherein said stems (6) comprise respective flexible reinforcing cores (2).

4. A prosthesis according to claim 2, wherein said cores (2) comprise string.

5. A prosthesis according to claim 2, wherein said spacer (3) and said stems (6) comprise one piece of said elastomeric material.

6. A prosthesis according to claim 5, wherein said spacer (3) and said stems (6) comprise a flexible reinforcing core (2).

7. A prosthesis according to claim 6, wherein said one piece has been moulded with said core (2) therein.

8. A prosthesis according to claim 6 or 7, wherein said core (2) comprises string.

9. A prosthesis according to any preceding claim, wherein said spacer (3) is of spherical shape but with flat end faces (4) from which said stems (6) extend.

## Patentansprüche

1. Prothesengelenk mit einem Abstandsstück (3), das zwischen einem ersten und zweiten Knochenteil eingefügt wird, und mit einem ersten und zweiten ins Knochenmark eingreifenden Schaft (6), die sich von entsprechenden einander gegenüberliegenden Enden des Abstandsstücks (3) zur Einführung in die Knochen erstrecken, wobei das Abstandsstück (3) eine größere radiale Ausdehnung als die Schäfte (6) und dadurch Schultern (4) aufweist, gegen die sich die jeweiligen Enden der Knochen anlegen können dadurch gekennzeichnet, daß bei eingesetzter Prothese das Gelenk sich in den dem Abstandsstück (3) nahen Bereichen der Schäfte (6) verbiegt.

2. Prothese nach Anspruch 1, dadurch gekennzeichnet, daß die Schäfte (6) aus elastomerem Material bestehen.

3. Prothese nach Anspruch 1 oder 2, dadurch gekennzeichnet, daß die Schäfte (6) jeweils flexible Verstarkungskerne (2) aufweisen.

4. Prothese nach Anspruch 2 dadurch gekennzeichnet, daß die Kerne (2) aus Schnüren bestehen.

5. Prothese nach Anspruch 2 dadurch gekennzeichnet, daß das Abstandsstück (3) und die Schäfte (6) einstückig aus elastomerem Material bestehen.

6. Prothese nach Anspruch 5 dadurch gekennzeichnet, daß das Abstandsstück (3) und die Schäfte (6) einen flexiblen Verstärkungskern (2) einschließen.

7. Prothese nach Anspruch 6 dadurch gekennzeichnet, daß dieses einstückige Teil mit darin eingeschlossenem Kern (2) ausgeformt wird.

8. Prothese nach Anspruch 6 oder 7 dadurch gekennzeichnet, daß der Kern (2) aus Litzen besteht.

9. Prothese nach einem der vorhergehenden Ansprüche dadurch gekennzeichnet, daß das Abstandsstück (3) eine Kugelform mit an den Seiten abgeflachten Endflächen (4) aufweist, von denen die Schäfte (6) vorragen.

**Revendications**

1. Prothèse articulaire, comprenant une entretoise (3) devant s'insérer entre les premier et second os et les première et les seconde tiges intramédullaires (6) s'étendant à partir des extrémités opposées respectives de ladite entretois (3) pour s'insérer dans les os respectifs, l'entretoise (3) étant plus large que les tiges (6); formant ainsi des épaulements (4) contre lesquels peuvent venir buter les extrémités respectives des os, caractérisée par une disposition telle que, dans la prothèse, une fois celle-ci implantée, la flexion de l'articulation se produise dans les zones d'extrémités respectives de la tige (6) proche de l'entretoise (3).

2. Prothèse articulaire suivant la revendication 1 où lesdites tiges (6) comportent une matière élastomère.

3. Prothèse suivant les revendications 1 ou 2, où lesdites tiges (6) comportent des noyaux centraux de renfort flexibles respectifs (2).

4. Prothèse suivant la revendication 2, où lesdits noyaux centraux (2) comportent un fil.

5. Prothèse suivant la revendication 2, où ladite entretoise (3) et lesdits tiges (6) comportent une pièce de ladite matière élastomère.

6. Prothèse suivant la revendication 5, où ladite entretoise (3) et lesdites tiges (6) comportent un noyau central de renfort flexible (2).

7. Prothèse suivant la revendication 6, où ladite pièce a été moulée avec ledit noyau (2) à l'intérieur.

8. Prothèse suivant les revendications 6 ou 7, où ledit noyau (2) comprend un fil.

9. Prothèse suivant l'une quelconque des revendications précédentes, où ladite entretoise (3) est de forme sphérique, mais avec des surfaces d'extrémités plates (4) à partir desquelles s'étendent lesdites tiges.